# EUROPEAN PATENT APPLICATION

(11) **EP 1 366 767 A1**
(43) Date of publication of application: **03.12.2003**
(21) Application number: 02712376.9
(22) Date of filing: 15.02.2002
(51) Int. Cl.: A61K 31/522, A61K 9/10, A61K 47/32, A61P 31/12, A61P 27/02

(54) **AQUEOUS SUSPENSION EYEDROPS AND PROCESS FOR PRODUCING THE SAME**

(30) Priority: 16.02.2001 JP 2001039437
(71) Applicant: NOF CORPORATION, Tokyo 150-6019 (JP); Toa Pharmaceutical Co., Ltd., Toyama-shi, Toyama 939-3548 (JP)
(72) Inventor: SAITOH, Kouichi, Amagasaki-shi, Hyogo 661-0012 (JP); KISHIMOTO, Yoko, Akashi-shi, Hyogo 674-0081 (JP); TOKUYAMA, Satoru, Nishinomiya-shi, Hyogo 663-8184 (JP); OGURO, Susumu, Nakaniikawa-gun, Toyama 930-0241 (JP); HANAZOME, Isao, Toyama-shi, Toyama 931-8443 (JP); OKAMOTO, Tomoyuki, Toyama-shi, Toyama 939-8073 (JP); KASAMA, Toshio, Toshima-ku, Tokyo 171-0031 (JP)
(74) Representative: Albrecht, Thomas, Dr.
(86) International application number: JP0201285
(87) International publication number: WO02064144

(57) **Abstract**

An ophthalmic aqueous suspension preparation to serve as a cure for corneal herpes infection contains 9-[(2-hydroxyethoxy)methyl]guanine as an active ingredient and exhibits an improved stability. Specifically, the ophthalmic aqueous suspension preparation contains 0.2 to 5% by weight of 9-[(2-hydroxyethoxy)methyl]guanine or a pharmaceutically acceptable salt thereof, 0.05 to 1% by weight of a partially saponified product of polyvinyl acetate, and 0.01 to 1% by weight of a nonionic surfactant and has a pH of 6 to 8. Preferably, the fine crystals have a short axis of 50µm or less and a long axis of 70µm or less in length and have a ratio of the long axis to the short axis of 10:1 or less.

## Description

### TECHNICAL FIELD

The present invention relates to an ophthalmic aqueous suspension preparation that contains as an active ingredient 9-[(2-hydroxyethoxy)methyl]guanine, a hardly water-soluble antiviral agent, or a pharmaceutically acceptable salt thereof. The present invention also relates to a method for producing such a preparation.

### TECHNICAL BACKGROUND

9-[(2-hydroxyethoxy)methyl]guanine, commonly known as acyclovir (JAN; INN) (which may be referred to simply as acyclovir, hereinafter), is an antiviral agent with a purine skeleton that selectively acts against herpes viruses while exhibiting little cytotoxicity against normal cells. It is one of the most widely used clinical compounds for treating herpes infections.

Acyclovir has a very low solubility in water: it can dissolve in an acidic or basic aqueous solution but hardly dissolve in an aqueous solution of near neutral pH. Though acyclovir can dissolve to some extent in an aqueous solution under acidic or basic condition, these solutions tend to be unstable and readily form crystals. For this reason, it has been difficult to develop stable aqueous acyclovir preparations that can be stored for a long time. Thus, the only form of ophthalmic acyclovir preparations currently in clinical use against corneal herpes infection is ointment and no ophthalmic preparation has ever been provided in the form of aqueous solution.

Several drawbacks exist for such ophthalmic acyclovir ointments. Patients often have difficulty applying such an ointment by themselves. Even if successfully applied, the ointment impedes the vision of the patients immediately after application and it usually takes several hours before they regain normal vision. Thus, to respond to a need for ophthalmic preparations that can be easily applied by patients, several types of preparations have thus far been proposed.

For example, Japanese Patent Laid-Open Publication No. Hei 8-268892 describes an ophthalmic preparation provided in the form of an aqueous solution in which polyvinylpyrrolidone is blended with acyclovir. Also, an antiviral composition containing acyclovir and a polyol is proposed in Japanese Patent Laid-Open Publication No. Hei 9-143096. Each of these preparations, however, requires the use of some solubilizer, such as polyvinylpyrrolidone and polyol, in order to enhance solubility of otherwise hardly water-insoluble acyclovir. Though effective to some degree, these solubilizers must be added in limited amounts and the dissolved acyclovir still shows some tendency to crystallize. These preparations are therefore insufficient in terms of the stability, especially when the preparations need to be stored for a prolonged time.

Unlike the above-described ophthalmic preparations provided in the form of aqueous solution, several ophthalmic preparations have been provided in the form of suspension. For example, an ophthalmic preparation described in Japanese Patent Laid-Open Publication No. Hei 10-287552 is a sterile aqueous suspension of acyclovir, which is prepared by suspending acyclovir in water, heating and then filtering the suspension. A suspension disclosed in Japanese Patent Laid-Open Publication No. Hei 11-228386 is prepared by dissolving a hardly water-soluble active ingredient, such as acyclovir, in an aqueous medium under basic condition, and subsequently adding an acid to allow crystals to form, at which time an ion-sensitive, hydrophilic polymer to serve as a thickener is added to form a suspension. Another suspension preparation disclosed in Japanese Patent Laid-Open Publication No. 2000-247887 is formed by first completely dissolving acyclovir in aqueous medium at a pH of 2 or below in the presence of hydrophilic polymer and subsequently adjusting the pH of the solution to a value of 6 to 9 to allow acyclovir to crystallize, thereby making the solution into a suspension.

The above-described preparations are each prepared in the form of suspension in order to compensate for the low solubility of acyclovir. In each case, acyclovir is first completely dissolved in aqueous medium by heating or adjusting the pH and is subsequently recrystallized to form crystals with the size suitable for ophthalmic application.

None of these suspension preparations, however, have succeeded in providing acyclovir crystals that remain stable when suspended in aqueous medium: though the suspensions are effective to some extent in that they can contain increased amounts of acyclovir to serve as the active ingredient, it is only at the time of the preparation of the suspension that the crystals of hardly water-soluble acyclovir exist in a properly small size and changes will eventually take place in the state of the crystals over the course of a long-term storage period. Specifically, the growth of the acyclovir crystals cannot be avoided in any of the above-described preparations. Accordingly, these suspension preparations are less than satisfactory for use as a pharmaceutical preparation.

Despite the expectation that if successfully developed, a simple anti-viral ophthalmic preparation containing acyclovir as the active ingredient should serve as a highly effective cure for the corneal herpes infection, no such preparation has ever been put to practical use. Thus, a need exists for developing an effective ophthalmic preparation that contains a sufficient amount of acyclovir while maintaining stability required for pharmaceutical products.

The study by the present inventors has revealed that it is not only when a solution in which acyclovir has been dissolved is cooled that the growth of crystals takes place, but also when the hardly water-soluble compound is suspended in aqueous medium in the form of fine crystals. When subjected to changes in temperature or exposed to a dispersing agent present in the suspension, the suspended crystals of acyclovir grow relatively rapidly to form needle crystals or plate crystals that are several tens to 100µm in length and have a ratio of the long axis to the short axis of more than 10:1.

Since the growth of crystals is unfavorable in ophthalmic aqueous suspension preparations, the present inventors have made an effort to find a way to prevent this from taking place and to thereby make a stable aqueous suspension preparation of acyclovir.

As a result, the present inventors have discovered that the growth of crystals can be effectively prevented without losing the bioavailability of acyclovir by suspending, rather than dissolving, crystals of acyclovir in aqueous medium in the pH range of 3 to 8 in the presence of both a partially saponified product of polyvinyl acetate and a nonionic surfactant, and subsequently adjusting the pH to a value of 6 to 8, the optimum pH range for ophthalmic preparations. The present inventors have found that in this manner, a pharmacologically stable ophthalmic preparation can be obtained in the form of aqueous suspension. This finding ultimately led the present inventors to devise the present invention.

### DISCLOSURE OF THE INVENTION

Accordingly, it is an objective of the present invention to provide an aqueous suspension preparation for ophthalmic use that contains as an active ingredient a therapeutically effective amount of acyclovir and has a high stability required of a pharmaceutical product. It is another objective of the present invention to provide a method for producing such a stable ophthalmic suspension preparation.

To this end, one aspect of the present invention provides a method for producing an ophthalmic acyclovir preparation in the form of a stable aqueous suspension. Specifically, the invention of claim 1 is a method for producing an ophthalmic aqueous suspension preparation comprising the steps of providing an aqueous solution that has a pH of 3 to 8 and contains 0.05 to 1% by weight of a partially saponified product of polyvinyl acetate and 0.01 to 1% by weight of a nonionic surfactant, suspending fine crystals of acyclovir or a pharmaceutically acceptable salt thereof in the aqueous solution in an amount of 0.2 to 5% by weight, and subsequently adjusting the pH of the suspension to a value of 6 to 8.

The invention of claim 2 is a method for producing an ophthalmic aqueous suspension preparation comprising the steps of providing an aqueous solution that has a pH of 3 to 8 and contains 0.05 to 1% by weight of a partially saponified product of polyvinyl acetate, suspending fine crystals of acyclovir or a pharmaceutically acceptable salt thereof in the aqueous solution in an amount of 0.2 to 5% by weight, adjusting the pH of the suspension to a value of 6 to 8, adding 0.01 to 1% by weight of a nonionic surfactant, and subsequently stirring the suspension.

In essence, what is characteristic of the method of the present invention is that fine crystals of acyclovir or a pharmaceutically acceptable salt thereof are suspended, but not completely dissolved, in aqueous medium in the presence of both a partially saponified product of polyvinyl acetate and a nonionic surfactant and in a predetermined pH range. In this manner, the crystals of acyclovir or a pharmaceutical salt thereof can be effectively prevented from growing and, as a result, an ophthalmic preparation can be obtained in the form of a stable aqueous suspension.

In this respect, the invention of claim 3 is the method in accordance with claim 1 or 2, wherein the degree of saponification of the partially saponified product of polyvinyl acetate is in the range of 70 to 95mol%. The use of the partially saponified polyvinyl acetate having a degree of saponification in the specified range can help prevent the growth of the fine crystals of acyclovir or a pharmaceutically acceptable salt in the suspension.

The invention of claim 4 is the method in accordance with claim 1 or 2, wherein the degree of polymerization of the partially saponified product of polyvinyl acetate is in the range from 300 to 2000. The use of the partially saponified polyvinyl acetate having a degree of polymerization in the specified range can more effectively help prevent the growth of the fine crystals of acyclovir or a pharmaceutically acceptable salt in the suspension.

The invention of claim 5 is the method in accordance with claim 1 or 2, wherein the suspension further contains 0.01 to 1% by weight of polyvinylpyrrolidone. The addition of polyvinylpyrrolidone in the specified amount facilitates dispersion of acyclovir or a pharmaceutically acceptable salt thereof to serve as the active ingredient of the suspension preparation.

Another aspect of the present invention provides an ophthalmic preparation containing acyclovir in the form of a stable aqueous suspension. Specifically, the invention of claim 6 is an ophthalmic aqueous suspension preparation produced by one of the above-described methods for producing an ophthalmic aqueous suspension preparation.

Preferably, the fine crystals of acyclovir or a pharmaceutically acceptable salt thereof suspended in the ophthalmic aqueous suspension preparation of the present invention have a particle size that allows acyclovir to exert desired therapeutic effects but does not prevent the active ingredient from migrating to humor aqueous.

The invention of claim 7 is the aqueous suspension preparation in accordance with claim 6, wherein the fine crystals of acyclovir or a pharmaceutically acceptable salt thereof suspended in the preparation has a short axis of 50µm or less and a long axis of 70µm or less in length. The ratio of the long axis to the short axis is preferably 10:1 or less.

### BRIEF DESCRIPTION OF THE DRAWINGS

Fig. 1 is a phase contrast micrograph showing fine crystals of acyclovir used in preparing an aqueous suspension preparation of the present invention.
Fig. 2 is a differential interference micrograph showing Preparation 8 (Example) prepared in Examples of the present invention, in which (2-a) and (2-b) show the preparation after a one-day and a one-month storage period at room temperature, respectively.
Fig. 3 is a phase contrast micrograph showing Preparation 12 (Example) one day following its preparation in Examples of the present invention.
Fig. 4 is a phase contrast micrograph showing Preparation 25 (Comparative Example) prepared in Comparative Examples of the present invention, in which (4-a) and (4-b) show the preparation after a one-day and a one-month storage period at room temperature, respectively.
Fig. 5 is a phase contrast micrograph showing Preparation 32 (Comparative Example) one day following its preparation in Comparative Examples of the present invention.

### BEST MODE FOR CARRYING OUT THE INVENTION

Ophthalmic preparation provided in the form of aqueous suspension in accordance with the present invention will now be described in further detail.

An active ingredient for use in the ophthalmic aqueous suspension preparation of the present invention is acyclovir or a pharmaceutically acceptable salt thereof. Examples of the pharmaceutically acceptable salt of acyclovir include salts with an inorganic acid, such as hydrochloric acid, sulfuric acid, and phosphoric acid; salts with an organic acid, such as acetic acid, lactic acid, citric acid, fumaric acid, maleic acid, and succinic acid; and salts with an alkali metal or an alkali earth metal, such as sodium, potassium, and calcium.

Hereinafter, the term "acyclovir" refers to acyclovir itself and a pharmaceutically acceptable salt thereof. In other words, the term is meant to encompass, in addition to free acyclovir, any pharmaceutically acceptable salt of acyclovir.

Preferably, fine crystals of acyclovir to serve as the active ingredient in the preparation of the present invention have a particle size that allows the active ingredient to exert desirable therapeutic effects without impeding the migration of the suspended acyclovir crystals to humor aqueous since the aqueous suspension preparation is intended for ophthalmic use. For that purpose, fine crystals are preferred that are sized such that the short axis and the long axis of the crystal each have a length of 75µm or less and the ratio of the long axis to the short axis is 10:1 or less. In particular, the fine crystals are preferred that have a short axis length of 50µm or less and a long axis length of 70µm or less and have a ratio of the long axis to the short axis of 10:1 or less. Fine crystals having such a particle size are stable over a prolonged time and can be used in preparing a desired aqueous suspension.

The fine crystals of acyclovir are preferably contained in the suspension in an amount of 0.2 to 5% by weight and more preferably, in an amount of 0.5 to 3% by weight. The amount of the fine crystals that is less than 0.2% by weight cannot provide sufficient acyclovir in the preparation and may be too small an amount to ensure the desired pharmacological effects. In contrast, the fine crystals of hardly water-soluble acyclovir, when present in an amount exceeding 5% by weight, tend to form aggregates in the suspension. This can result in a decreased ability of the suspension to prevent the growth of the crystals and, thus, in a reduced stability of the final product of the suspension preparation.

The partially saponified product of polyvinyl acetate for use in the aqueous suspension preparation of the present invention is a hydrophilic polymer obtained by polymerizing vinyl acetate and then saponifying some of the acetyl groups present in the polyvinyl acetate molecule. The compound may be referred to as a partially saponified polyvinyl alcohol. In this case, "polyvinyl alcohol" means a polyol in which most of the acetyl groups present in the polyvinyl acetate have been substituted for hydroxyl groups, and it turned out that, for the purpose of preparing the aqueous suspension preparation of the present invention, a partially saponified product of polyvinyl acetate with a degree of saponification of 70 to 95mol% is preferred.

If the degree of saponification is lower than 70mol%, then the desired effect of preventing the growth of crystals of acyclovir may not be achieved by the addition of the partially saponified product of polyvinyl acetate. Moreover, the polyvinyl acetate product becomes susceptible to hydrolysis to generate acetic acid. As a result, the stability of the final product of the aqueous suspension preparation may be reduced. Conversely, if the degree of saponification is higher than 95mol%, then the other ingredients added to the ophthalmic preparation (e.g., boric acid and sodium borate) may cause the preparation to undergo gelation.

The partially saponified product of polyvinyl acetate for use in the present invention is typically added in an amount of 0.05 to 1% by weight and preferably, in an amount of 0.1 to 1% by weight. The amount that is less than 0.05% by weight may result in a decreased ability of the partially saponified product of polyvinyl acetate to suppress the growth of crystals and is thus unfavorable. On the other hand, the amount exceeding 1% by weight may cause excessively high viscosity of the aqueous suspension preparation. As a result, acyclovir to serve as the active ingredient tends to form aggregates, resulting in a decreased ability of the suspension preparation to suppress the growth of crystals. Furthermore, the ease at which user can apply the ophthalmic preparation is significantly affected.

The partially saponified product of polyvinyl acetate for use in the present invention typically has a degree of polymerization of 300 to 2000, preferably 300 to 1700, and more preferably 300 to 1400. With the degree of polymerization of less than 300, the partially saponified product may not provide sufficient ability to prevent the growth of crystals. On the other hand, the degree of polymerization of greater than 2000 may result in an excessively high viscosity of the suspension preparation. As a result, acyclovir may form aggregates, which in turn may result in a decreased ability of the suspension preparation to suppress the growth of crystals. Furthermore, the ease at which user can apply the ophthalmic preparation is significantly affected.

For typical partially saponified products of polyvinyl acetate currently available in the marketplace, the degree of polymerization (JIS K6726) is measured by the viscosity of a 4% aqueous solution measured at 20°C. The partially saponified product of polyvinyl acetate for use in the present invention preferably has a degree of polymerization as measured the viscosity of 3 to 60mPa·s and more preferably, 3 to 40mPa·s.

The aqueous suspension preparation of the present invention, which can be prepared by suspending fine crystals of acyclovir to serve as the active ingredient in aqueous medium, requires a nonionic surfactant as a dispersing agent to facilitate the dispersion of the fine crystals.

The nonionic surfactants for use in the present invention may be those that are selected for its safety from conventional nonionic surfactants used in pharmaceutical products. Examples include polysorbates (polyoxyethylenesorbitan mono-fatty acid esters), polyoxyethylene castor oil, polyoxyethylene hardened castor oil, polyoxyl stearates, and polyoxyethylene polyoxypropylene glycols.

In the present invention, the nonionic surfactants that exhibit a relatively high hydrophilicity are preferred because of their high ability to enhance the dispersion of acyclovir crystals and in view of their use for ophthalmic application. For example, those having an HLB (hydrophile-lipophile balance) of 10 or more, preferably 12 or more, are preferably used.

Examples of the nonionic surfactant include polysorbate 80, polyoxyethylene hardened castor oil 40, polyoxyethylene hardened castor oil 60, polyoxyl stearate 40, polyoxyethylene (160) polyoxypropylene (30) glycol, and, polyoxyethylene (200) polyoxypropylene (70) glycol. Of these, polysorbate 80 and polyoxyethylene hardened castor oil 60 are particularly preferred in view of their use for ophthalmic application.

The nonionic surfactant is typically added in an amount of 0.01 to 1% by weight and, preferably, in an amount of 0.05 to 0.5% by weight. If the amount of the nonionic surfactant is less than 0.01% by weight, then acyclovir to serve as the active ingredient may not be stably suspended, which may result in a reduced stability of the final product of the suspension preparation. In contrast, the nonionic surfactant, when added in an amount exceeding 1% by weight, raises the concentration of the surfactant in the preparation, causing the fine crystals of acyclovir to form aggregates. As a result, the stability of the dispersion may be decreased.

In essence, the aqueous suspension preparation of the present invention comprises fine crystals of acyclovir, the active ingredient, suspended in an aqueous solution that contains the partially saponified product of polyvinyl acetate and the nonionic surfactant. Thus, should the fine crystals of acyclovir to serve as the active ingredient be precipitated, the preparation may be lightly shaken upon use to redisperse the acyclovir crystals. It has been found that the fine crystals of acyclovir, if precipitated, do not affect the quality of the product.

However, the suspension preparation in accordance with the present invention further incorporates polyvinylpyrrolidone as a means to facilitate redispersion of the precipitated fine crystals of acyclovir to serve as the active ingredient. Polyvinylpyrrolidone to be added to enhance the redispersion of acyclovir crystals may be selected from those that are commonly in use in pharmaceutical applications and may have a molecular weight of about 20000 to about 50000.

Polyvinylpyrrolidone is typically added to the suspension preparation in an amount of 0.01 to 1% by weight and, preferably, in an amount of 0.1 to 1% by weight. If the amount is less than 0.01% by weight, then the desired effect of enhancing re-dispersal of the fine crystals of acyclovir may not be achieved by the addition of polyvinylpyrrolidone. Conversely, the amount exceeding 1% by weight may cause excessively high viscosity of the final product of the suspension preparation. As a result, acyclovir may form aggregates, resulting in a decreased ability of the suspension preparation to suppress the growth of crystals. Too much polyvinylpyrrolidone also impedes the redispersion of the acyclovir crystals.

While the generally accepted pH range of aqueous ophthalmic preparations that does not cause eye irritation is from 4.8 to 8.5, the purpose of adjusting the pH of ophthalmic preparation is mainly to ensure the stability of the drug preparations and to enhance the efficacy of the drug, rather than to reduce eye irritation (Guidelines for Drug Formulation, 10th ed., Japan Pharmaceutical Association ed.,).

In the aqueous suspension preparation of the present invention, it is also preferred to adjust the pH to a near neutral range acceptable for ophthalmic preparations. Specifically, the pH of the aqueous suspension preparation of the present invention is preferably adjusted to a range of 6 to 8 in consideration of the stability of the fine crystals of acyclovir to serve as the active ingredient.

If the suspension preparation has a pH of below 6 or above 8, the solubility of the fine crystals of acyclovir to serve as the active ingredient is subject to change, so that the growth of crystals is more likely to occur. Furthermore, the partially saponified product of polyvinyl acetate contained in the preparation becomes less stable, as does the nonionic surfactant. As a result, the stability of the suspension preparation may be reduced.

As long as the stability of the pH is ensured, the aqueous suspension preparation of the present invention may further contain various other components that are commonly used in ophthalmic preparations, including an isotonizing agent, a buffer, a preservative, and a pH adjuster.

Examples of the isotonizing agent include water-soluble polyols, such as glycerol, propylene glycol, sorbitol and mannitol; and electrolytes, such as sodium chloride and potassium chloride. Of these, glycerol is particularly preferred.

Aside from boric acid and a mixed system of boric acid/sodium borate (sodium tetraborate) that are widely used in ophthalmic preparations, examples of the buffer include a mixed system of sodium dihydrogenphosphate/disodium hydrogenphosphate, and a mixed system of potassium dihydrogenphosphate/disodium hydrogenphosphate.

Other acids or alkalis other than the aforementioned boric acid (or a salt thereof) and phosphoric acid (or a salt thereof), such as hydrochloric acid and sodium hydroxide, may also be used to serve as the pH adjuster.

Examples of the preservative include quaternary ammonium salts such as benzalkonium chloride and benzethonium chloride, chlorhexidine gluconate, alkyldiaminoethylglycine hydrochloride, parabens such as methyl p-hydroxybenzoate and propyl p-hydroxybenzoate, and chlorobutanol. One or more of these compounds are properly selected for use. The amount of the preservative to be added is from about 0.003 to 0.02% by weight for quaternary ammonium salt, from about 0.05 to 0.1% by weight for paraben, and from about 0.25 to 0.5% by weight for chlorobutanol.

A specific method for producing the aqueous suspension preparation of the present invention will now be described.

In producing the aqueous suspension preparation of the present invention, fine crystals of acyclovir, a hardly water-soluble antiviral agent, are suspended and dispersed, at a specific pH range, in a solution that contains a predetermined amount of a partially saponified product of polyvinyl acetate along with a predetermined amount of a nonionic surfactant. The fine crystals of acyclovir are suspended in the solution without being completely dissolved. In this manner, a stable ophthalmic suspension preparation containing acyclovir can be obtained.

As long as this method is applied, other conditions are not limited. However, the following two-step method is preferably used to produce a stable aqueous suspension preparation containing fine crystals of acyclovir.

The first step of the preferred method for producing aqueous ophthalmic suspension preparation of the present invention comprises suspending fine crystals of acyclovir in a solution that contains the partially saponified product of polyvinyl acetate and has a pH in the range of 3 to 8, preferably in the range of 4 to 7.5.

If the solution has a pH of below 3, the solubility of the fine crystals of acyclovir in water increases. Thus, when the pH is later adjusted to a near neutral range, the solubility of acyclovir drops significantly, so that the fine crystals of acyclovir become more likely to grow into a needle shape during the pH adjustment. As a result, the stability of the crystals in the final product of the aqueous suspension preparation may be undesirably decreased.

Similarly, if the solution has a basic pH of above 8, the solubility of the fine crystals of acyclovir in water increases. Thus, when the pH is later adjusted to a near neutral range, the solubility of acyclovir drops significantly, so that the fine crystals of acyclovir become more likely to grow into a needle shape during the pH adjustment. Moreover, the acetyl groups present in the partially saponified product of polyvinyl acetate become susceptible to saponification. Thus, again, the stability of the crystals in the final product of the aqueous suspension preparation may be undesirably decreased.

In the first step of suspending the fine crystals of acyclovir, the size of the fine crystals is reduced to 40µm or less, preferably to 30µm or less, when acyclovir having 75µm or more of crystal size is used for the preparation. In this manner, the resulting suspension preparation can be made more suitable for ophthalmic application. Conditions for stirring for suspending acyclovir in the solution are properly selected depending on the particle size of acyclovir used.

The second step of the method of the present invention comprises re-adjusting the pH of the suspension prepared in the first step to a pH range of 6 to 8, preferably to a pH range of 6.5 to 7.5, adding the nonionic surfactant and, if necessary, polyvinylpyrrolidone, and further stirring the suspension. In this manner, the final product of aqueous suspension preparation is obtained.

If the final product of the suspension preparation has a pH of below 6 or above 8, then the desired effect of suppressing the growth of crystals by the partially saponified product of polyvinyl acetate may not be obtained and the growth of the fine crystals of acyclovir may take place. Also, the preparation with such a pH can cause eye irritation upon application.

In the above-described method of producing the aqueous suspension preparation of the present invention, the nonionic surfactant and optional polyvinylpyrrolidone may be added during the second step unless the desired stability of the aqueous suspension preparation is affected. Alternatively, they may be added, together with the partially saponified product of polyvinyl acetate, during the first step.

To ensure the pH stability of the final product of the aqueous suspension preparation in the method of the present invention, it is preferred to use an acid such as boric acid or sodium dihydrogenphosphate to prepare the solution with a pH of 3 to 8 used in the first step in which to suspend the fine crystals of acyclovir. When it is desired to adjust the pH of the suspension to an acidic pH in the first step and then adjust the pH to 6 to 8 in the second step, a base commonly used in adjusting the pH of ophthalmic preparations may suitably be used. By taking into account the pH stability of the final product of the aqueous suspension preparation, it is desirable to use sodium hydroxide or sodium borate, as well as disodium hydrogenphosphate, which can be used in combination with the aforementioned sodium dihydrogenphosphate to impart a buffering property to the aqueous suspension preparation.

Alternatively, a buffer solution, such as a phosphate buffer and a borate buffer, that has had its pH preadjusted by combining the above-described acid and base may be used in the first step.

In the method for producing the aqueous suspension preparation of the present invention, the additional components such as the preservative and the isotonizing agent may be added in a proper manner in either of the first and the second steps.

For example, when it is desired to use the preservative, the amount of the component is adjusted depending on the property of the selected preservative. Also, when it is desired to use the isotonizing agent, the amount of the component is adjusted so that the final preparation has an osmotic pressure of 250mOsm to 400mOsm with the total osmotic pressure brought about by the other components taken into account.

The aqueous suspension preparation obtained by the method of the present invention, which is designed for ophthalmic application, needs to be presterilized. This is done by using a proper technique selected from those that are generally applicable to sterilization of pharmaceutical products. Specifically, since the aqueous suspension preparation of the present invention is provided in the form of suspension in which acyclovir to serve as the active ingredient has been suspended, the final preparation cannot be sterilized by heating or filtration. For this reason, it is desirable to independently sterilize the fine crystals of acyclovir and other components by proper techniques prior to preparation of the suspension.

In the event that heat is generated during the stirring/dispersing step from a stirrer/disperser used in the first or the second step, it is desirable to implement the process while properly cooling these instruments. Such stirring/dispersing instruments may be properly selected from those that are generally used in pharmaceutical application, including disperser, homomixer, and homogenizer. Of these, those instruments that can efficiently reduce the size of the solid (crystal) suspended in the solution to a desired crystal size are particularly preferred. Specific examples include rotary homogenizers, such as Clearmix (manufactured by Cleartech Co., Ltd.), wet type jet mills, and high-pressure jet stream emulsifiers.

As set forth, according to the method for producing an aqueous suspension preparation of the present invention, a highly stable aqueous suspension preparation in which the growth of fine crystals of acyclovir to serve as the active ingredient is significantly suppressed can be obtained by first suspending fine crystals of acyclovir in an aqueous solution that has a pH ranging from acidic to neutral and contains a partially saponified product of polyvinyl acetate and a nonionic surfactant; and subsequently adjusting the pH of the suspension to a pH of 6 to 8, at which acyclovir exhibits minimum solubility in water and which is close to the pH of lacrimal fluid.

The aqueous suspension preparation prepared by the method of the present invention has an advantage that, in the event that the suspended fine crystals of acyclovir are precipitated during storage, the crystals can be readily resuspended by applying a light shake and, thus, the need to increase the viscosity of the preparation for the purpose of maintaining the stability of the preparation has been eliminated. Accordingly, the aqueous suspension preparation of the present invention can be applied just in the same manner as other common ophthalmic preparations without causing unpleasant feelings to eyes.

### EXAMPLES

The present invention will now be described in detail with reference to Examples, which are not intended to limit the scope of the invention in any way.

### 1. Examples/Comparative Examples (Production of aqueous suspension preparation containing acyclovir)

### I) Formulation

Different aqueous suspension preparations containing acyclovir (Examples/Comparative Examples) were prepared according to the formulations shown in Tables 1-1 through 1-5.

**Table 1-1:**

| Compositions of aqueous suspension preparations (w/v%) | | | | | | | | |
|---|---|---|---|---|---|---|---|---|
| Preparation No. | Examples | | | | | | | |
| | Prep. 1 | Prep. 2 | Prep. 3 | Prep. 4 | Prep. 5 | Prep. 6 | Prep. 7 | Prep. 8 |
| Acyclovir¹⁾ | 1.0 | 1.0 | 1.0 | 2.0 | 2.0 | 2.0 | 3.0 | 3.0 |
| [Partially saponified product of polyvinyl acetate] GOHSENOL GL-05 | 0.2 | 0.2 | - | - | - | - | 0.5 | 0.5 |
| GOHSENOL GM-14 | - | - | 0.1 | 0.5 | 0.5 | - | - | - |
| GOHSENOL GH-20 | - | - | - | - | - | 0.2 | - | - |
| [Disperser] Polyvinylpyrrolidone K-25 | - | 0.25 | - | - | 0.5 | - | - | 0.5 |
| [Nonioic surfactant] polysorbate 80 | 0.1 | 0.1 | - | 0.3 | 0.3 | - | 0.5 | 0.5 |
| polyoxyethylene hardened castor oil 60 | - | - | 0.1 | - | - | 0.3 | - | - |
| [Buffer] | 50mM phosphate buffer²⁾ | | | | | | | |
| [Isotonizing agent] | Glycerol | | | | | | | |
| [Preservative] | Methylparaben/propylparaben | | | | | | | |
| pH measured immediately after preparation | 6.47 | 6.40 | 6.51 | 6.42 | 6.38 | 6.46 | 6.35 | 6.32 |
| Osmotic pressure (mOsm) | 291 | 305 | 288 | 299 | 301 | 292 | 297 | 309 |

| | | | | | | | | |
|---|---|---|---|---|---|---|---|---|
| 1) Acyclovir: As measured by the amount of solid component. | | | | | | | | |
| 2) Sodium dihydrogenphosphate/disodium hydrogenphosphate buffer(pH 6.5) | | | | | | | | |

**Table 1-2:**

| Compositions of aqueous suspension preparations (w/v%) | | | | | | | | |
|---|---|---|---|---|---|---|---|---|
| Preparation No. | Examples | | | | | | | |
| | Prep. 9 | Prep. 10 | Prep. 11 | Prep. 12 | Prep. 13 | Prep. 14 | Prep. 15 | Prep. 16 |
| Acyclovir¹⁾ | 3.0 | 3.0 | 3.0 | 3.0 | 3.0 | 3.0 | 3.0 | 3.0 |
| [Partially saponified product of polyvinyl acetate] GOHSENOL KP-06 | 0.5 | - | - | - | - | - | - | - |
| GOHSENOL KL-05 | - | 0.5 | - | - | - | - | - | - |
| GOHSENOL KH-17 | - | - | 0.5 | - | - | - | - | - |
| GOHSENOL EG-05 | - | - | - | 0.5 | 1.0 | - | - | 1.0 |
| GOHSENOL EG-25 | - | - | - | - | - | 0.5 | - | - |
| GOHSENOL GL-05 | - | - | - | - | - | - | 0.5 | - |
| [pH adjuster 1] | 50mM aqueous solution of sodium dihydrogenphosphate³⁾ | | | | | | | |
| pH upon suspending | 4.0 | 4.0 | 4.2 | 4.1 | 4.2 | 4.0 | 4.2 | 4.2 |
| [pH adjuster 2] | 1N aqueous solution of sodium hydroxide⁴⁾ | | | | | | | |
| [Nonioic surfactant] Polysorbate 80⁵⁾ | 0.3 | 0.3 | 0.3 | 0.3 | 0.3 | 0.3 | 0.3 | 0.3 |
| [Isotonizing agent] | Glycerol⁵⁾ | | | | | | | |
| [Preservative] | Benzalkonium chloride | | | | | | | |
| pH measured immediately after preparation | 6.72 | 6.92 | 7.22 | 6.85 | 6.97 | 6.67 | 6.82 | 7.36 |
| Osmotic pressure (mOsm) | 307 | 313 | 313 | 305 | 317 | 308 | 311 | 321 |

| | | | | | | | | |
|---|---|---|---|---|---|---|---|---|
| 1) Acyclovir: As measured by the amount of solid component. | | | | | | | | |
| 3) Used to suspend acyclovir. | | | | | | | | |
| 4) Used to adjust pH after acyclovir was suspended. | | | | | | | | |
| 5) Added following the addition of pH conditioner 2 | | | | | | | | |

**Table 1-3:**

| Compositions of aqueous suspension preparations (w/v%) | | | | | | | | |
|---|---|---|---|---|---|---|---|---|
| Preparation No. | Ex. | Comparative Examples | | | | | | |
| | Prep. 17 | Prep. 18 | Prep. 19 | Prep. 20 | Prep. 21 | Prep. 22 | Prep. 23 | Prep. 24 |
| Acyclovir¹⁾ | 2.0 | 2.0 | 2.0 | 2.0 | 2.0 | 2.0 | 2.0 | 2.0 |
| [Hydrophilic polymer] GOHSENOL GL-05 | 0.5 | - | - | - | - | - | - | - |
| GOHSENOL NH-26 | - | 0.5 | - | - | - | - | - | - |
| Hydroxypropyl methylcellulose | - | - | 0.3 | - | - | - | - | - |
| Polyvinyl pyrrolidone K-25 | - | - | - | -0.5 | 2.0 | - | - | - |
| Polyethyleneglycol 4000 | - | - | - | - | - | 1.0 | - | - |
| Carboxyvinylpolymer (Carbopole 941⁶⁾) | - | - | - | - | - | - | 0.03 | - |
| Carboxyvinylpolymer (Carbopole 971P⁶⁾) | - | - | - | - | - | - | - | 0.03 |
| [Nonionic surfactant] Polysorbate 80 | 0.3 | 0.3 | 0.3 | 0.3 | 0.3 | 0.3 | 0.3 | 0.3 |
| [Buffer/pH adjuster] | 50mM phosphate buffer⁷⁾ | | | | | | 50mM phosphate buffer⁸⁾ | |
| [Isotonizing agent] | Glycerol | | | | | | | |
| [Preservative] | methylparaben/propylparaben | | | | | | | |
| pH measured immediately after preparation | 6.96 | 6.93 | 6.97 | 6.97 | 6.88 | 6.92 | 6.71 | 6.76 |
| Osmotic pressure (mOsm) | 313 | 304 | 314 | 302 | 318 | 300 | 294 | 291 |

| | | | | | | | | |
|---|---|---|---|---|---|---|---|---|
| 1) Acyclovir: As measured by the amount of solid component. | | | | | | | | |
| 6) Carbopole was dissolved in an aqueous solution of sodium hydroxide prior to use. | | | | | | | | |
| 7) Sodium dihydrogenphosphate/disodium hydrogenphosphate buffer (pH 7.0) | | | | | | | | |
| 8) Sodium dihydrogenphosphate/disodium hydrogenphosphate buffer (pH 7.0): Mixed with an aqueous solution of Carbopole. | | | | | | | | |

**Table 1-4:**

| Compositions of aqueous suspension preparations (w/v%) | | | | | | | | |
|---|---|---|---|---|---|---|---|---|
| Preparation No | Comparative Examples | | | | | | | |
| . | Prep. 25 | Prep. 26 | Prep. 27 | Prep. 28 | Prep. 29 | Prep. 30 | Prep. 31 | Prep. 32 |
| Acyclovir¹⁾ | 2.0 | 2.0 | 2.0 | 2.0 | 2.0 | 2.0 | 2.0 | 2.0 |
| [Hydrophilic polymer] GOHSENOL GL-05 | 0.5 | - | - | - | - | - | - | - |
| GOHSENOL NL-05 | - | 0.5 | - | - | - | 0.5 | - | - |
| Carboxyvinylpolymer (Carbopole 941) | - | - | 0.03 | - | - | - | 0.03 | - |
| Carboxyvinylpolymer (carbopole 971P) | - | - | - | 0.03 | - | - | - | 0.03 |
| [pH adjuster 1]⁹⁾ | 1N hydrochloric acid | | | | 1N aqueous solution of sodium hydroxide | | | |
| pH upon dissolving | 1.33 | 1.36 | 1.68 | 1.62 | 10.78 | 10.82 | 10.82 | 10.84 |
| [pH adjuster 2]¹⁰⁾ | 1N aqueous solution of sodium hydroxide | | | | 1N hydrochloric acid | | | |
| [Nonioic surfactant] Polysorbate 80¹¹⁾ | 0.3 | 0.3 | 0.3 | 0.3 | 0.3 | 0.3 | 0.3 | 0.3 |
| [Isotonizing agent] | Glycerol¹¹⁾ | | | | | | | |
| [Preservative] | Benzalkonium chloride | | | | | | | |
| pH measured immediately after preparation | 6.98 | 6.96 | 7.01 | 6.98 | 7.02 | 7.02 | 6.94 | 6.96 |
| Osmotic pressure (mOsm) | 298 | 297 | 292 | 290 | 293 | 291 | 297 | 298 |

| | | | | | | | | |
|---|---|---|---|---|---|---|---|---|
| 1) Acyclovir: As measured by the amount of solid component. | | | | | | | | |
| 9) Used to dissolve acyclovir. | | | | | | | | |
| 10) Used to adjust pH after acyclovir was completely dissolved. | | | | | | | | |
| 11) Added following the addition of pH adjuster 2 to adjust pH to a near neutral range. | | | | | | | | |

**Table 1-5:**

| Compositions of aqueous suspension preparations (w/v%) | | | | | | | | |
|---|---|---|---|---|---|---|---|---|
| Preparation No. | Comparative Examples | | | | | | | |
| | Prep. 33 | Prep. 34 | Prep. 35 | Prep. 36 | Prep. 37 | Prep. 38 | Prep. 39 | Prep. 40 |
| Acyclovir¹⁾ | 2.0 | 2.0 | 10.0 | 0.2 | 3.0 | 3.0 | 2.0 | 2.0 |
| [Hydrophilic polymer] GOHSENOL GL-05 | 0.02 | 5.0 | 1.0 | - | - | - | - | 0.5 |
| [Nonionic surfactant] Polysorbate 80 | 0.3 | 0.3 | 0.3 | 0.3 | 0.3 | 1.5 | - | - |
| [Buffer/pH adjuster] | 50mM phosphate buffer¹²⁾ | | | | | | | |
| [Isotonizing agent] | Glycerol | | | | | | | |
| [Preservative] | methylparaben/propylparaben | | | | | | | |
| pH measured immediately after preparation | 6.94 | 6.92 | 6.96 | 6.99 | 6.94 | 6.95 | 7.02 | 6.96 |
| Osmotic pressure (mOsm) | 302 | -* | 313 | 305 | 314 | 309 | 300 | 305 |

| | | | | | | | | |
|---|---|---|---|---|---|---|---|---|
| 1) Acyclovir: As measured by the amount of solid component. | | | | | | | | |
| 12) Sodium dihydrogenphosphate/disodium hydrogenphosphate buffer (pH 7.0) | | | | | | | | |
| *) Unable to determine (measured by osmometry). | | | | | | | | |

### II) Ingredients

The following ingredients were used:

### 1. Acyclovir

USP grade, sterilized acyclovir was used as fine crystals of acyclovir without any processing. Through laser diffraction particle size distribution analysis, the crystals were determined to have an average particle size of 25.9µm. The observation with a microscope revealed that no needle crystals were present that had a ratio of the long axis to the short axis of more than 10:1.

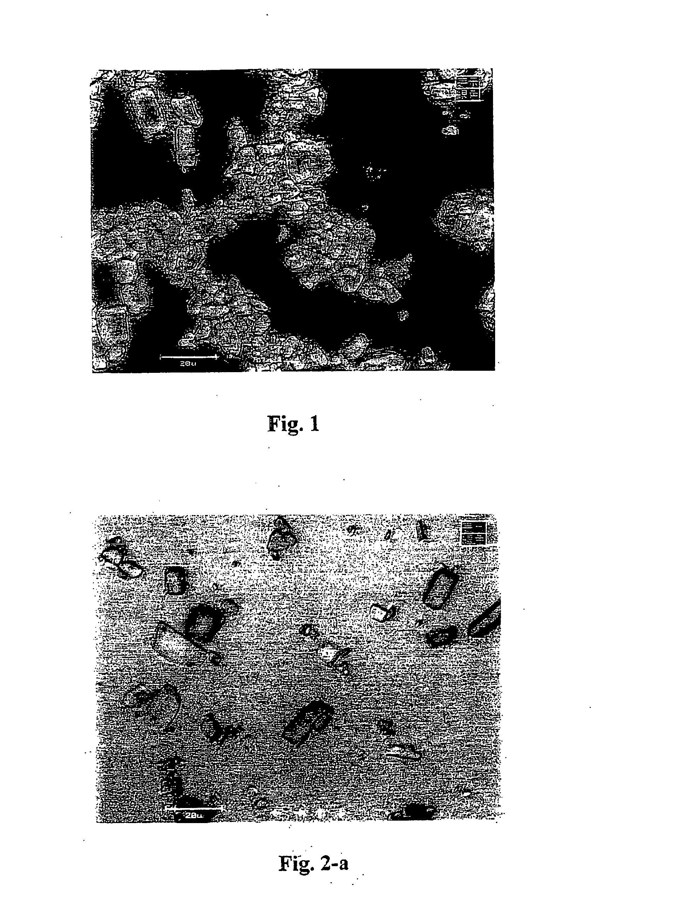

A phase contrast micrograph of the fine crystals of acyclovir used is shown in Fig. 1.

### 2. Partially saponified product of polyvinyl acetate

The degree of saponification, the degree of polymerization, and the viscosity (4% aqueous solution, 20°C) were shown in Table 2 below for each of the partially saponified products of vinyl acetate used to prepare the suspension preparations.

**Table 2:**

| Degree of saponification and degree of polymerization of partially or completely saponified products of polyvinyl acetate. | | | | |
|---|---|---|---|---|
| Partially saponified products of polyvinyl acetate | | Degree of saponification (mol%) | Degree of polymerization | Viscosity (mPa·s) |
| GOHSENOL GL-05 | NIPPON SYNTHETIC CHEMICAL INDUSTRY CO., LTD. | 87.8 | 500 | 5.4 |
| GOHSENOL GM-14 | | 87.7 | 1400 | 22.0 |
| GOHSENOL GH-20 | | 86.7 | 2000 | 42.0 |
| GOHSENOL KP-06 | | 72.4 | 600 | 6.5 |
| GOHSENOL KL-05 | | 79.9 | 500 | 4.4 |
| GOHSENOL KH-17 | | 79.3 | 1700 | 34.8 |
| GOHSENOL EG-05 | | 87.6 | 500 | 5.7 |
| GOHSENOL EG-25 | | 88.2 | 1400 | 22.5 |
| GOHSENOL NL-05 | | 99.1 | 500 | 5.0 |
| GOHSENOL NH-26 | | 99.4 | 2600 | 65.4 |

Additional two suspension preparations were prepared to serve as Comparative Examples: one containing a different hydrophilic polymer rather than the partially saponified product of polyvinyl acetate and one containing neither the partially saponified product of polyvinyl acetate nor hydrophilic polymer were prepared.

Each of the partially saponified products of polyvinyl acetate was measured by the amount of dehydrated solid component.

### 3. Nonionic surfactant

Polysorbate 80 (which meets the standard set by the Pharmacopoea of Japan) or polyoxyethylene hardened castor oil 60 (which meets the Standard for the Pharmaceutical Additives) was used to serve as the nonionic surfactant. For comparison, a suspension preparation that did not contain the surfactant was also prepared to serve as Comparative Example.

### 4. Other ingredients

Other ingredients, namely, buffer, pH adjuster, isotonizing agent and preservative, were as shown in Table 1.

### III) Production methods of suspension preparation

Each of the aqueous suspension preparations was prepared by using Clearmix CLM-0.8S (Product name: Manufactured by CREATECH Co., Ltd.), a rotary homogenizer.

Specifically, the suspension preparations were prepared according to the following two methods.

### 1) Method 1

For example, each of Preparations 1 through 8 was prepared in the following manner: First, to a 50mM phosphate buffer solution (pH 6.5) containing an effective amount of the preservative, one of the partially saponified products of polyvinyl acetate and the nonionic surfactant shown in Table 1-1 were added (in Preparations 2, 5, and 8, polyvinylpyrrolidone was further added). After the added ingredients were completely dissolved, the fine crystals of acyclovir were added and the mixture was isotonized with glycerol. Subsequently, the mixture was stirred for 10 minutes at 10,000rpm while being cooled with water to suspend acyclovir crystals and to thereby give an aqueous suspension preparation.

### 2) Method 2

For example, each of Preparations 9 through 16 was prepared in the following manner: To a 50mM aqueous solution of sodium dihydrogenphosphate containing an effective amount of the preservative, one of the partially saponified products of polyvinyl acetate shown in Table 1-2 was added. After completely dissolving the added ingredients, the fine crystals of acyclovir were added and the mixture was stirred for 5 minutes at 10,000rpm while being cooled with water to suspend the acyclovir crystals. The pH of the suspension at this point was determined to be 4.0 to 4.2. Using a 1N aqueous solution of sodium hydroxide, the pH of the suspension was then adjusted to 6.5 to 7.5. Subsequently, the nonionic surfactant shown in Table 1-2 was added and the suspension was isotonized with glycerol. This was followed by stirring for 5 minutes at 10,000rpm while the suspension was cooled with water. This gave an aqueous suspension preparation.

The two methods were properly modified to prepare the different aqueous suspension preparations that are formulated according to the compositions shown in Tables 1-1 through 1-5 above (Examples/Comparative Examples). The pH and the osmotic pressure of each of the aqueous suspension preparations were measured immediately after preparation of the suspension and are shown in respective Tables.

The osmotic pressure was measured using Advanced Osmometer Model 3D3 (manufactured by ADVANCED INTRUMENTS Co., Ltd.).

### 2. Tests for assessing the stability and other properties of the preparations

5ml aliquots were collected from each of the aqueous suspension preparations prepared above and were individually placed in a 5ml 3-pieced polyethylene eye drop bottle for testing.

### 1) Test for the stability of acyclovir crystal

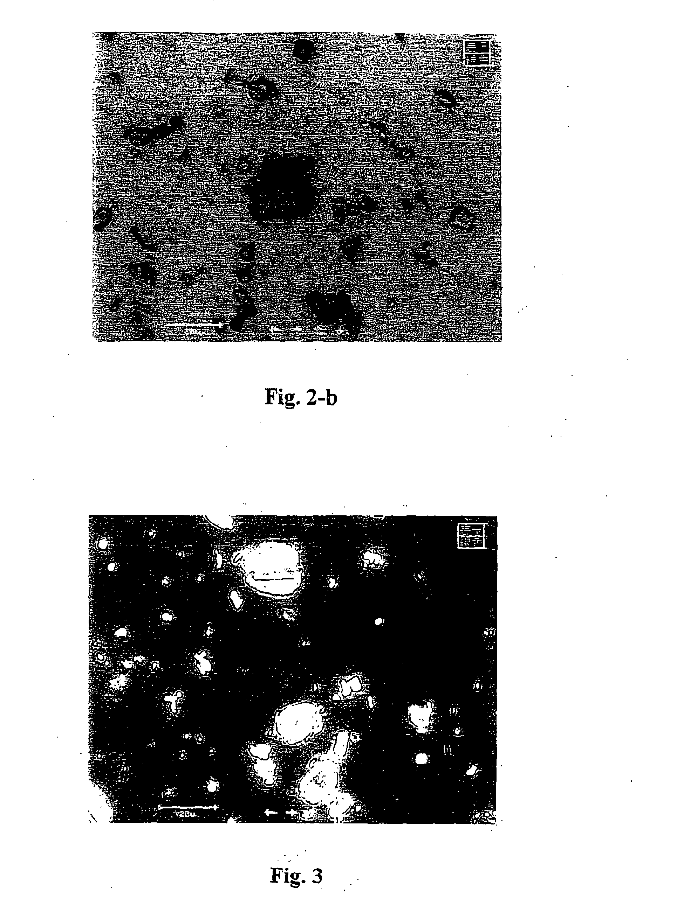

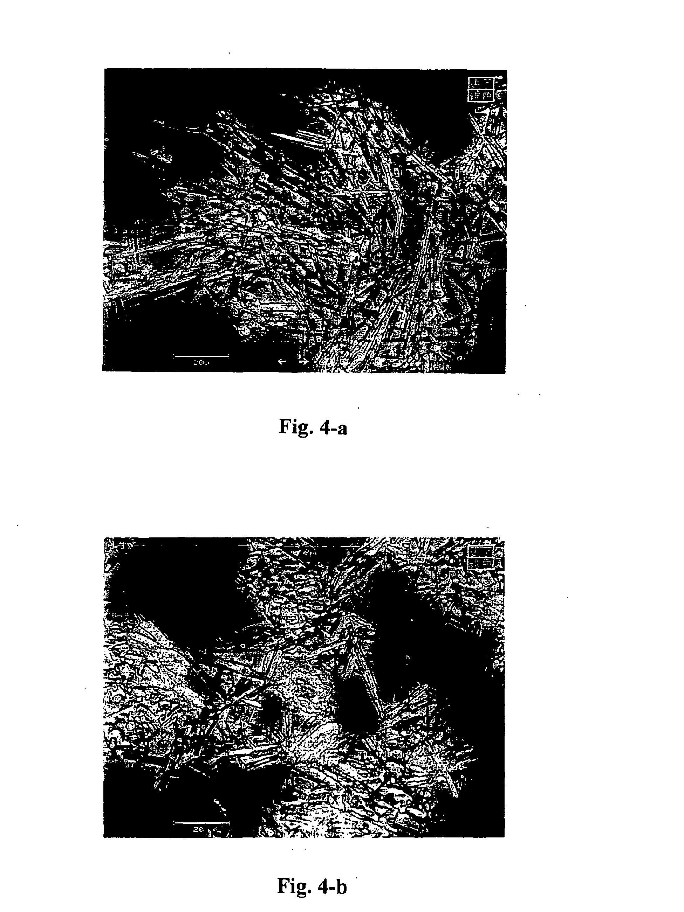

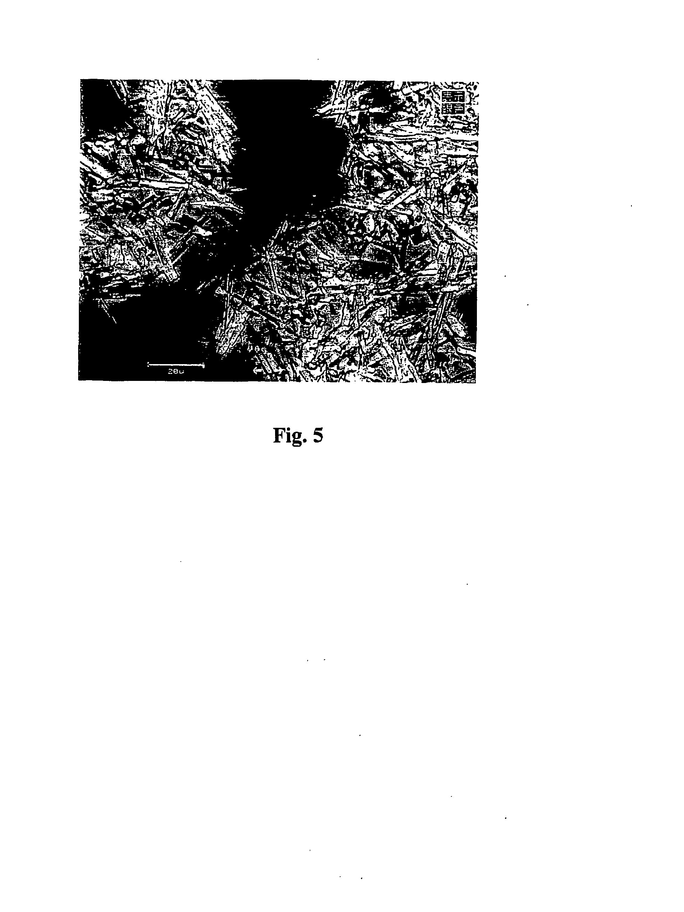

The aqueous suspension preparations were each sampled immediately after preparation of the suspensions and were observed for the conditions of the crystals through phase contrast microscopy or differential interference microscopy using a light microscope (BX50, manufactured by OLYMPUS Co., Ltd.). No growth of the suspended acyclovir crystals into needle crystals was observed in any of Preparations 1 through 24. In contrast, the formation of numerous needle crystals was observed immediately after preparation of the suspension in each of Preparations 25 through 32 (Comparative Examples), in which the fine crystals of acyclovir were first completely dissolved and later recrystallized through adjustment of pH. The state of the resultant crystals are shown in a differential interference micrograph or a phase contrast micrograph in Figs 2 through 5 for some of the representative Preparations.

Each of the aqueous suspension preparations prepared in Examples/Comparative Examples above was evaluated in the following manner for the stability of the resultant crystals: Vials containing respective suspension preparations were stored in a thermostatic chamber at room temperature and at 40°C, respectively. The vials were taken out of the chamber at time intervals and were each time shaken by hands to make the suspension uniform. Any change that took place in the fine crystals of acyclovir in each preparation was then observed by a phase contrast light microscope and was rated. The ratings were given as follows:

### Ratings:

Each sample was observed in 20 fields of view and were rated as follows:

A double circle (ⓞ) indicates that the growth of fine crystals into needle crystals was not observed, nor was the aggregation of the crystals.

A single circle (○) indicates that no needle crystals were found that have the ratio of the long axis to the short axis of more than 10:1, but the crystals aggregated to some degree.

A triangle (Δ) indicates that formation of needle or plate crystals with the ratio of the long axis to the short axis of more than 10:1 was observed in some of the observed fields of view.

A single cross (×) indicates that significant formation of needle or plate crystals with the ratio of the long axis to the short axis of more than 10:1 was observed in most of the observed fields of view.

A double cross (××) indicates that substantially all of the crystals grew to needle crystals having a ratio of the long axis to the short axis of 10:1 or more.

The degree of aggregation of the fine crystals of acyclovir was also rated as follows:

A hollow square (□) indicates that significant aggregation of crystals was observed in most of the observed fields of view.

A solid square (■) indicates that most of the crystals aggregated.

The lengths of the short axis and the long axis of the fine crystals of acyclovir were measured by processing micrographs of the crystals with an image processor (Model VM-30, manufactured by OLYMPUS Co., Ltd.). The results are summarized in Table 3 below.

**Table 3:**

| Changes in the state of fine crystals of acyclovir in aqueous suspension preparation | | | | | | |
|---|---|---|---|---|---|---|
| Prep. No | Changes in the state of crystals | | | | | |
| | Room | temperature | | 40°C | | |
| | 1 month | 3 month | 6 month | 1 month | 3 month | 6 month |
| 1 | ⓞ | ⓞ | ⓞ | ⓞ | ⓞ | ⓞ |
| 2 | ⓞ | ⓞ | ⓞ | ⓞ | ⓞ | ⓞ |
| 3 | ⓞ | ⓞ | ⓞ | ⓞ | ⓞ | ○ |
| 4 | ⓞ | ⓞ | ○ | ⓞ | ⓞ | ○ |
| 5 | ⓞ | ⓞ | ⓞ | ⓞ | ⓞ | ○ |
| 6 | ⓞ | ⓞ | ○ | ⓞ | ○ | □ |
| 7 | ⓞ | ⓞ | ⓞ | ⓞ | ⓞ | ⓞ |
| 8 | ⓞ | ⓞ | ⓞ | ⓞ | ⓞ | ⓞ |
| 9 | ⓞ | ⓞ | ⓞ | ⓞ | ⓞ | ⓞ |
| 10 | ⓞ | ⓞ | ⓞ | ⓞ | ⓞ | ⓞ |
| 11 | ⓞ | ⓞ | ○ | ⓞ | ⓞ | ○ |
| 12 | ⓞ | ⓞ | ⓞ | ⓞ | ⓞ | ⓞ |
| 13 | ⓞ | ⓞ | ⓞ | ⓞ | ⓞ | ⓞ |
| 14 | ⓞ | ⓞ | ⓞ | ⓞ | ⓞ | ○ |
| 15 | ⓞ | ⓞ | ⓞ | ⓞ | ⓞ | ⓞ |
| 16 | ⓞ | ⓞ | ⓞ | ⓞ | ⓞ | ⓞ |
| 17 | ⓞ | ⓞ | ⓞ | ⓞ | ⓞ | ⓞ |
| 18 | ⓞ | ○ | Δ | ⓞ | Δ | □ |
| 19 | ○ | Δ | Δ | ○ | Δ | Δ |
| 20 | ⓞ | ○ | Δ | ⓞ | ○ | Δ |
| 21 | ○ | Δ | □ | ○ | Δ | □ |
| 22 | Δ | Δ□ | ×■ | Δ | ×■ | ×■ |
| 23 | × | ××□ | ××□ | ××□ | ××□ | ××□ |
| 24 | × | ××□ | ××□ | ××□ | ××□ | ××□ |
| 25 | ××□ | ××□ | ××□ | ××□ | ××□ | ××□ |
| 26 | ××□ | ××□ | ××□ | ××□ | ××□ | ××□ |
| 27 | ××□ | ××□ | ××□ | ××□ | ××□ | ××■ |
| 28 | ××□ | ××□ | ××□ | ××□ | ××□ | ××■ |
| 29 | ××□ | ××□ | ××□ | ××□ | ××□ | ××□ |
| 30 | ××□ | ××□ | ××□ | ××□ | ××□ | ××□ |
| 31 | ××□ | ××□ | ××□ | ××□ | ××□ | ××■ |
| 32 | ××□ | ××□ | ××□ | ××□ | ××□ | ××■ |
| 33 | Δ | × | × | Δ | × | × |
| 34 | ○ | □ | ■ | ○ | ■ | Δ■ |
| 35 | Δ | Δ□ | Δ□ | Δ | Δ□ | ×□ |
| 36 | × | × | × | × | × | × |
| 37 | Δ | × | × | Δ | × | × |
| 38 | Δ | ×□ | ×□ | Δ | ×□ | ×□ |
| 39 | Δ | × | × | Δ | × | × |
| 40 | ⓞ | ○ | Δ | ○ | Δ | Δ |

As can be seen from the results of Table 3, fine crystals of acyclovir remained stable over time in the aqueous suspension preparations produced in accordance with the method of the present invention and the state of fine crystals present immediately after preparation of the suspension was maintained.

### 2) Test for the stability of aqueous suspension preparation

Each of the aqueous suspension preparations prepared in Examples/Comparative Examples above was evaluated in the following manner for the stability of the preparation: Eyedrop vials containing respective suspension preparations were stored in a thermostatic chamber at room temperature and at 40°C, respectively. The vials were taken out of the chamber at time intervals and the pH of each preparation was measured each time. The results are shown in Table 4 for some of the representative preparations obtained in Examples and Comparative Examples above.

**Table 4:**

| pH of aqueous suspension preparations | | | |
|---|---|---|---|
| Prep. No | pH | | |
| | after preparation | 3 months at RT. | 3 months at 40°C |
| 1 | 6.47 | 6.32 | 6.12 |
| 2 | 6.40 | 6.14 | 5.97 |
| 3 | 6.51 | 6.28 | 6.18 |
| 4 | 6.42 | 6.23 | 6.06 |
| 5 | 6.38 | 6.19 | 6.01 |
| 6 | 6.46 | 6.07 | 5.98 |
| 7 | 6.35 | 6.11 | 5.94 |
| 8 | 6.32 | 5.97 | 5.70 |
| 9 | 6.72 | 6.68 | 6.36 |
| 10 | 6.92 | 6.66 | 6.48 |
| 11 | 7.22 | 7.04 | 6.91 |
| 12 | 6.85 | 6.55 | 6.51 |
| 13 | 6.97 | 6.51 | 6.49 |
| 14 | 6.67 | 6.65 | 6.57 |
| 15 | 6.82 | 6.57 | 6.52 |
| 16 | 7.36 | 7.26 | 7.09 |
| 17 | 6.96 | 6.81 | 6.67 |
| 25 | 6.98 | 5.47 | 3.24 |
| 30 | 7.02 | 5.23 | 3.03 |

As can be seen from the results of Table 4, each of the aqueous suspension preparations obtained in accordance with the method of the present invention has proven pharmaceutically stable. In contrast, a significant decrease in pH was observed in the preparations of Comparative Examples (Preparations 25 and 30), indicating the lack of pharmaceutical stability. The amount and osmotic pressure of acyclovir contained in Preparations 1 to 17, each an aqueous suspension preparation of the present invention, were also measured over time. No significant changes were observed in the amount or the osmotic pressure of the acyclovir during the observation period of the test. With all of the measurements taken of these factors falling within the permissive range, each of the preparations has proven stable.

### 3) Test for the redispersibility of acyclovir fine crystals

Of the different aqueous suspension preparations prepared in Examples above, Preparations 1, 2, Preparations 4, 5, and Preparations 7, 8, and 17 were examined for the redispersibility of the precipitated fine crystals of acyclovir: Vials containing respective suspension preparations were stored at room temperature and were inverted multiple times at time intervals to disperse the fine crystals uniformly. The number of times that a vial had to be inverted before the crystals become uniform was determined. The ratings were given as follows:

A double circle (ⓞ) indicates that the vial containing the preparation was inverted 10 times or less to redisperse the crystals.

A single circle (○) indicates that the vial had to be inverted 10 to 20 times to redisperse the crystals.

A triangle (Δ) indicates that the vial had to be inverted 20 to 30 times to redisperse the crystals.

A cross (×) indicates that the vial had to be inverted 30 to 40 times to redisperse the crystals.

A double cross (××) indicates that the vial had to be inverted over 40 times to redisperse the crystals.

The results are shown in Table 5.

**Table 5**

| Dispersibility of aqueous suspension preparation | | | |
|---|---|---|---|
| Prep.No | Dispersibility | | |
| | 1 month later | 3 month later | 6 month later |
| 1 | ⓞ | ⓞ | ○ |
| 2 | ⓞ | ⓞ | ⓞ |
| 4 | ○ | ○ | ○ |
| 5 | ⓞ | ⓞ | ○ |
| 7 | ○ | ○ | ○ |
| 8 | ⓞ | ⓞ | ⓞ |
| 17 | ⓞ | ○ | ○ |

As can be seen from the results of Table 5, the addition of polyvinylpyrrolidone according to the method of the present invention significantly improved the redispersibility of the precipitated fine crystals of acyclovir (Preparations 2, 5, and 8).

### 2. Test for the migration of acyclovir to humor aqueous

Of the different aqueous suspension preparations prepared in Examples above, Preparations 2, 4, 8, 12, and 16 were examined for the migration of acyclovir to humor aqueous after application to eyes: 50µL of each of the acyclovir-containing aqueous suspension preparations prepared in Examples were applied to eyes of 4 rabbits (New Zealand White rabbits, male, weighing 2.0kg to 3.0kg). After 1 hour, the animals were locally anesthetized with benoxyl and all of the humor aqueous was collected from each animal with a 26G needle.

A standard solution was also prepared for each of the preparations applied to the rabbits. Using HPLC, the amount of acyclovir present in the humor aqueous was determined, as was the amount of acyclovir in the standard.

The results of the test were shown for each preparation in Table 6 below.

**Table 6:**

| Migration of acyclovir in the suspension preparation to humor aqueous | |
|---|---|
| Prep. No | Conc. of acyclovir in humor aqueous (µg/mL) (mean ± SD) |
| 2 | 0.11±0.05 |
| 4 | 0.22±0.08 |
| 8 | 0.28±0.07 |
| 12 | 0.19±0.02 |
| 16 | 0.25±0.06 |

As shown in Table 6, the concentration of acyclovir in humor aqueous, which is indicative of degree of migration of acyclovir to humor aqueous, was significantly higher than a value of approximately 0.03µg/mL, the ID₅₀ (50% inhibition level) of acyclovir against herpes virus (Crumpaker, C. S. *et al., Antimicrob. Agents Chemother.,* 15, 642 (1979)), for each of the aqueous suspension preparation. Thus, each preparation was determined to be effective as an ophthalmic preparation.

### INDUSTRIAL APPLICABILITY

As set forth, the aqueous ophthalmic suspension preparation of the present invention, which contains acyclovir, a hardly water-soluble antiviral agent, to serve as the active ingredient, is characterized in that it is formed by suspending a predetermined amount of fine crystals of acyclovir in an aqueous solution containing a partially saponified product of polyvinyl acetate and a nonionic surfactant. This process is carried out at a predetermined pH range so that the crystals remain undissolved in the solution. In this manner, an ophthalmic preparation can be obtained in the form of pharmaceutically stable aqueous suspension in which the growth of acyclovir crystals is substantially prevented without compromising the efficacy of the drug.

This simple aqueous suspension of the present invention, which contains a sufficient amount of acyclovir while being capable of remaining pharmaceutically stable for a prolonged time period, makes a significant contribution to the current state of field of medicine wherein no effective ophthalmic preparations for treating corneal herpes infection has ever existed.

## Claims

1. A method for producing an ophthalmic aqueous suspension preparation, comprising the steps of providing an aqueous solution that has a pH of 3 to 8 and contains 0.05 to 1% by weight of a partially saponified product of polyvinyl acetate and 0.01 to 1% by weight of a nonionic surfactant, suspending fine crystals of acyclovir or a pharmaceutically acceptable salt thereof in the aqueous solution in an amount of 0.2 to 5% by weight, and subsequently adjusting the pH of the suspension to a value of 6 to 8.

2. A method for producing an ophthalmic aqueous suspension preparation comprising the steps of providing an aqueous solution that has a pH of 3 to 8 and contains 0.05 to 1% by weight of a partially saponified product of polyvinyl acetate, suspending fine crystals of 9-[(2-hydroxyethoxy)methyl]guanine or a pharmaceutically acceptable salt thereof in the aqueous solution in an amount of 0.2 to 5% by weight, adjusting the pH of the suspension to a value of 6 to 8, adding 0.01 to 1% by weight of a nonionic surfactant, and subsequently stirring the suspension.

3. The method according to claim 1 or 2, wherein a degree of saponification of the partially saponified product of polyvinyl acetate is in the range of 70 to 95mol%.

4. The method according to claim 1 or 2, wherein a degree of polymerization of the partially saponified product of polyvinyl acetate is in the range of 300 to 2000.

5. The method according to claim 1 or 2, wherein the suspension further contains 0.01 to 1% by weight of polyvinyl pyrrolidone.

6. An ophthalmic aqueous suspension preparation obtained by the method of claim 1 or 2.

7. The ophthalmic aqueous suspension preparation according to claim 6, wherein the suspended fine crystals of 9-[(2-hydroethoxy)methyl]guanine or the salt thereof have a short axis of 50µm or less and a long axis of 70µm or less in length and have a ratio of the long axis to the short axis of 10:1 or less.
